(19) Europäisches Patentamt
European Patent Office
Office européen des brevets

(11) **EP 3 274 014 B1**

(12) **EUROPÄISCHE PATENTSCHRIFT**

(45) Veröffentlichungstag und Bekanntmachung des Hinweises auf die Patenterteilung:
**24.04.2024   Patentblatt 2024/17**

(21) Anmeldenummer: **16711267.1**

(22) Anmeldetag: **21.03.2016**

(51) Internationale Patentklassifikation (IPC):
**A61M 1/16** (2006.01)

(52) Gemeinsame Patentklassifikation (CPC):
**A61M 1/1635; A61M 1/1647; A61M 1/1662; A61M 1/1664; A61M 1/3622;** A61M 1/3623; A61M 2205/127; A61M 2205/3334; A61M 2205/3372; A61M 2205/366

(86) Internationale Anmeldenummer:
**PCT/EP2016/056152**

(87) Internationale Veröffentlichungsnummer:
**WO 2016/150916 (29.09.2016 Gazette 2016/39)**

(54) **TEMPERATURSTÖRUNGSUNABHÄNGIGE BILANZIERUNGSEINRICHTUNG**

BALANCING DEVICE INDEPENDENT OF TEMPERATURE DISTURBANCE

SYSTÈME PERMETTANT D'ÉTABLIR UN BILAN INDÉPENDAMMENT DE LA TEMPÉRATURE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.03.2015   DE 102015104431**

(43) Veröffentlichungstag der Anmeldung:
**31.01.2018   Patentblatt 2018/05**

(73) Patentinhaber: **Fresenius Medical Care Deutschland GmbH**
**61352 Bad Homburg (DE)**

(72) Erfinder:
• **ABEL, Petra**
**61169 Friedberg (DE)**
• **HEIDE, Alexander**
**65817 Eppstein (DE)**
• **NIKOLIC, Dejan**
**65824 Schwalbach am Taunus (DE)**
• **PETERS, Arne**
**61352 Bad Homburg (DE)**
• **SCHULTE, Elke**
**97422 Schweinfurt (DE)**
• **WIKTOR, Christoph**
**63571 Gelnhausen (DE)**

(74) Vertreter: **Bobbert & Partner Patentanwälte PartmbB**
**Postfach 1252**
**85422 Erding (DE)**

(56) Entgegenhaltungen:
EP-A1- 0 715 859      EP-A1- 2 532 999
WO-A1-2013/175547    DE-A1-102010 003 642
US-A- 4 530 759

**Beschreibung**

Die vorliegende Erfindung betrifft eine medizinische Behandlungsvorrichtung gemäß Anspruch 1, eine medizinische Funktionseinrichtung gemäß Anspruch 5 und eine Bilanzierungseinrichtung gemäß Anspruch 10.

In der Medizin, insbesondere im Bereich der Blutbehandlung, beispielsweise der Dialyse, werden dem Patienten Flüssigkeiten zugeführt und/oder dem Patienten Flüssigkeiten entzogen (siehe z.B. US4530759 und EP0715859). Die genaue Bilanzierung dieser dem Patienten zugeführten bzw. entzogenen Flüssigkeitsmengen ist von besonderer Bedeutung für die Sicherheit und Gesundheit des Patienten.

Zur Bilanzierung werden in der Praxis Bilanzierungseinrichtungen verwendet. Diese können mit Blutbehandlungsvorrichtungen, beispielsweise Dialysevorrichtungen, verbunden sein.

Eine Aufgabe der vorliegenden Erfindung ist es, ein weiteres Bilanzierungsverfahren zur Bilanzierung von Flüssigkeitsströmen und eine weitere Bilanzierungseinrichtung anzugeben. Ferner sollen eine medizinische Funktionseinrichtung, eine medizinische Behandlungsvorrichtung, ein digitales Speichermedium, ein Computerprogramm sowie ein Computerprogramm-Produkt angegeben werden.

Die erfindungsgemäße Aufgabe wird durch Vorrichtungen mit den Merkmalen der Ansprüche 1, 5 und 10 gelöst.

Das hier offenbarte Verfahren betrifft somit das Bestimmen wenigstens einer Fluidbilanz zwischen wenigstens einem ersten Volumenstrom, insbesondere eines ersten Abschnitts eines Fluidkreislaufs einer Funktionseinrichtung, und wenigstens einem zweiten Volumenstrom, insbesondere eines zweiten Abschnitts des Fluidkreislaufs der Funktionseinrichtung.

Das Verfahren betrifft alternativ oder ergänzend das Steuern und/oder Regeln (beide Begriffe werden im Folgen der besseren Lesbarkeit halber als "Steuern" bezeichnet, was optional aber auch ein Regeln umfasst) wenigstens eines Volumenstroms wenigstens eines Abschnitts des Fluidkreislaufs der Funktionseinrichtung.

Das Verfahren umfasst wenigstens den Schritt des Einstellens, Erfassens und/oder Annehmens wenigstens eines ersten Volumenstroms in wenigstens einem ersten Abschnitt des Fluidkreislaufs. Das Verfahren weist ferner wenigstens den Schritt auf, wenigstens eine erste Temperatur in wenigstens einem ersten Abschnitt des Fluidkreislaufs einzustellen, anzunehmen oder zu Erfassen.

Das Verfahren umfasst ferner wenigstens den Schritt des Einstellens, Erfassens und/oder Annehmens wenigstens einer zweiten Temperatur in wenigstens einem zweiten Abschnitt des Fluidkreislaufs oder des Einstellens, Erfassens und/oder Annehmens wenigstens einer Temperaturdifferenz zwischen dem ersten und dem zweiten Abschnitt des Fluidkreislaufs.

Das Verfahren umfasst weiterhin wenigstens den Schritt des Einstellens, Erfassens und/oder Annehmens wenigstens eines zweiten Volumenstroms in wenigstens einem zweiten Abschnitt des Fluidkreislaufs.

Das Verfahren weist zudem wenigstens das Bilden einer Fluidbilanz aus wenigstens dem ersten Volumenstrom und einem korrigierten Wert des zweiten Volumenstroms auf. Der korrigierte Wert F2_korr wird wenigstens aus dem, insbesondere erfassten, zweiten Volumenstrom und der zweiten Temperatur und/oder der Temperaturdifferenz ermittelt und/oder berechnet.

Erfindungsgemäß wird ferner eine Funktionseinrichtung, welche vorzugsweise eine medizinische Funktionseinrichtung ist, vorgeschlagen. Sie umfasst wenigstens einen Fluidkreislauf, oder wenigstens einen Abschnitt hiervon oder ist hiermit verbunden und/oder verbindbar. Sie umfasst ferner wenigstens einen ersten und einen zweiten Durchflusssensor. Alternativ ist sie konfiguriert und/oder ausgebildet, um mit einem Durchflusssensor, insbesondere lösbar, verbunden und/oder verbindbar zu sein.

Dabei ist der Durchflusssensor zum Messen wenigstens eines Volumenstroms in wenigstens einem ersten und/oder in einem zweiten Abschnitt des Fluidkreislaufs, ausgebildet und/oder angeordnet.

Die Funktionseinrichtung weist ferner wenigstens eine Einrichtung zum Erfassen einer Temperatur im ersten und zweiten Abschnitt und/oder einer Temperaturdifferenz zwischen dem ersten und zweiten Abschnitt des Fluidkreislaufs auf. Eine solche Einrichtung kann in wenigstens einem Abschnitt des Fluidkreislaufs umfasst, angeordnet und/oder ausgebildet sein, insbesondere in einem ersten und/oder zweiten Abschnitt. Eine solche Einrichtung kann zum Messen der Temperatur wenigstens eines den Abschnitt durchströmenden oder sich darin befindenden Fluids und/oder zum Messen der Temperatur des jeweiligen Abschnitts vorgesehen und/oder ausgebildet sein.

Die erfindungsgemäße Bilanzierungseinrichtung ist vorgesehen und/oder ausgebildet zum Bestimmen wenigstens einer Fluidbilanz, insbesondere zwischen wenigstens oder genau einem ersten Volumenstrom in einem ersten Abschnitt eines Fluidkreislaufs und einem zweiten Volumenstrom in einem zweiten Abschnitt des Fluidkreislaufs.

Die Bilanzierungseinrichtung weist zu diesem Zweck wenigstens eine erfindungsgemäße Funktionseinrichtung auf. Ergänzend hierzu kann die Bilanzierungseinrichtung in Datenverbindung stehen mit wenigstens einer Einrichtung zum Erfassen der Temperatur, der Temperaturdifferenz und/oder des Volumenstroms des Fluidkreislaufs, oder mit wenigstens einem Teil der Funktionseinrichtung, insbesondere mit wenigstens einem Durchflusssensor und/oder wenigstens einer Pumpe, beispielsweise einer Blutpumpe.

Die erfindungsgemäße Bilanzierungseinrichtung weist die erfindungsgemäße Funktionseinrichtung auf.

Die erfindungsgemäße medizinische Behandlungsvorrichtung, welche zum Behandeln eines medizinischen Fluids ausgebildet und/oder konfiguriert ist, ist konfiguriert zum Ausführen des offenbarten Verfahrens.

Vorteilhafte Weiterbildungen der vorliegenden Erfindung sind jeweils Gegenstand der Unteransprüche.

Erfindungsgemäße Ausführungsformen können einige oder alle der folgenden Merkmale in beliebiger Kombination aufweisen.

Bei allen oben gemachten und bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung können jeweils auch Gegenstand der Unteransprüche sein.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann nicht erkennbar technisch unmöglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

Wann immer hierin die Rede von "geeignet", "vorgesehen", "ausgelegt", "konfiguriert" und/oder "programmiert" ist, kann der Fachmann dies als eine besondere Ausgestaltung der betreffenden Vorrichtung verstehen. Die vorgenannten Begriffe können hier austauschbar verwendet werden.

Obgleich die Erfindung im Folgenden vornehmlich anhand der Dialysebehandlung beschrieben wird, ist die Erfindung hierauf nicht beschränkt.

Der Begriff "Volumenstrom", wie er hierin verwendet wird, kann als das Volumen eines Fluids, das sich innerhalb eines gegebenen Zeitraum durch einen Querschnitt, beispielsweise des Fluidkreislaufs, bewegt hat, bezogen auf den hierfür erforderlichen Zeitraum, verstanden werden. Ein Volumenstrom kann beispielsweise in ml pro Minuten oder pro Stunde angegeben werden.

Der Begriff "Massenstrom", wie er hierin verwendet wird, kann gleich der Dichte des Fluids mal dem Volumenstrom sein.

Der Begriff "Erfassen", wie er hierin verwendet wird, kann allgemein ein Messen, ein Ermitteln, ein Lesen eines, beispielsweise gespeicherten und/oder eingestellten Wertes und/oder ein Berechnen, insbesondere aus bekannten, gespeicherten, erfassten und/oder eingestellten Werten, sein.

In manchen erfindungsgemäßen, beispielhaften Ausführungsformen wird das Verfahren im Zusammenwirken mit einer erfindungsgemäßen Funktionseinrichtung, einer erfindungsgemäßen Bilanzierungseinrichtung und/oder einer erfindungsgemäßen Blutbehandlungsvorrichtung ausgeführt.

In einigen erfindungsgemäßen, beispielhaften Ausführungsformen umfasst das Verfahren wenigstens das Einstellen oder Annehmen wenigstens einer Temperatur in wenigstens einem Abschnitt des Fluidkreislaufs. Es umfasst in diesen Ausführungsformen vorzugsweise wenigstens das Einstellen wenigstens eines Volumenstroms in wenigstens einem Abschnitt des Fluidkreislaufs.

Unter einem Annehmen einer Temperatur kann das Abschätzen der Temperatur oder das Festsetzen eines Wertes für die Temperatur für weitere Betrachtungen verstanden werden. Dies kann beispielsweise erfolgen, wenn der Wert der Temperatur aus Erfahrungswerten geschlossenen werden kann, etwa basierend auf der Temperatur, mit welcher das Fluid in einer Fluidquelle vorliegt oder diese verlässt.

Unter einem "Einstellen einer Temperatur" kann das Verändern der Temperatur, beispielsweise mittels Regler, Heizer, usw. verstanden werden.

In manchen erfindungsgemäßen, beispielhaften Ausführungsformen werden wenigstens die Werte des eingestellten Volumenstroms und/oder der eingestellten Temperatur erfasst, zumindest zeitweise gespeichert, oder hinterlegt.

In bestimmten erfindungsgemäßen, beispielhaften Ausführungsformen wird als Verfahrensschritt wenigstens ein Volumenstrom in einem Abschnitt des Fluidkreislaufs, beispielsweise ein erstes Volumenstrom in einem ersten Abschnitt, insbesondere vor Bildung der Fluidbilanz, eingestellt.

Unter "wenigstens ein Volumenstrom" bzw. "wenigstens einen Temperatur" kann hierhin beispielsweise der erste und/oder der zweite Volumenstrom, also der Volumenstrom des ersten und/oder des zweiten Abschnitts, bzw. die erste und/oder die zweite Temperatur, also die Temperatur des ersten und/oder des zweiten Volumenstroms, verstanden werden.

In manchen erfindungsgemäßen, beispielhaften Ausführungsformen kann die Fluidbilanz (hier als FB abgekürzt), der korrigierte Wert des Volumenstroms F2_korr, die Temperatur T1, T1' oder die Temperaturdifferenz DT als Signal und/oder zur Erzeugung eines Signals für eine Steuer- und/oder eine Regeleinrichtung zum Steuern und/oder Regeln wenigstens eines Volumenstroms des Fluidkreislaufs, einer Pumprate wenigstens einer Pumpe des Fluidkreislaufs, eines Querschnitts wenigstens eines Abschnitts des Fluidkreislaufs und/oder einer Temperatur im Fluidkreislauf, beispielsweise mittels wenigstens eines Heizers, verwendet werden.

In einigen erfindungsgemäßen, beispielhaften Ausführungsformen kann eine Fluidbilanz FB wenigstens aus einem ersten Volumenstrom F1 und einem zweiten Volumenstrom F2 gebildet werden.

In manchen erfindungsgemäßen, beispielhaften Ausführungsformen kann die Fluidbilanz FB folgendermaßen gebildet werden:

$$(\text{Formel 1}): \quad FB = F1 - F2,$$

oder

$$(\text{Formel 1'}): \quad FB = F2 - F1.$$

In einigen erfindungsgemäßen, beispielhaften Ausführungsformen wird eine Fluidbilanz FB aus wenigstens einem ersten Volumenstrom F1 oder F2 und einem korrigierten Wert eines zweiten Volumenstroms F2_korr oder F1_korr gebildet.

In manchen erfindungsgemäßen, beispielhaften Ausführungsformen kann die Fluidbilanz FB folgendermaßen gebildet werden:

$$(\text{Formel 2}): \quad FB = F1 - F2\_korr,$$

oder

$$(\text{Formel 2'}): \quad FB = F2\_korr - F1,$$

oder

$$(\text{Formel 2''}): \quad FB = F2 - F1\_Korr,$$

oder

$$(\text{Formel 2'''}): FB = F1\_Korr - F2.$$

In manchen erfindungsgemäßen, beispielhaften Ausführungsformen wird der korrigierte Volumenstrom oder Durchfluss-Wert F2_korr oder F1_korr wenigstens aus einem erfassten Volumenstrom F1 oder F2 und einer erfassten Temperatur T1, T1', T2 und/oder T2' ermittelt oder berechnet.

In einigen erfindungsgemäßen, beispielhaften Ausführungsformen kann der korrigierte Wert Fi_korr, mit i=1 oder 2 folgendermaßen gebildet werden:

$$(\text{Formel 3}): \quad Fi\_korr = Fi * (1 + K * (T1-T2)),$$

oder

$$(\text{Formel 3'}): \quad Fi\_korr = Fi * (1 + K * (T2-T1)),$$

wobei K ein Korrekturfaktor ist.

Der Korrekturfaktor K kann vorbestimmt sein. Er kann experimentell oder theoretisch ermittelt sein. Er kann gespeichert vorliegen. Er kann beispielsweise die Einheit [°C$^{-1}$] aufweisen. Er kann negativ, positiv sein. T1 und/oder T2 sind optional gemessene, erfasste, eingestellte oder ermittelte Temperaturwerte des Fluids in den jeweiligen Abschnitten des Fluidkreislaufs.

In manchen erfindungsgemäßen, beispielhaften Ausführungsformen wird der korrigierte Volumenstrom oder Durchfluss-Wert Fi_korr unter Berücksichtigung der Temperaturdifferenz DT zwischen zwei unterschiedlichen Abschnitten des Fluidkreislaufs ermittelt und/oder berechnet.

In gewissen erfindungsgemäßen, beispielhaften Ausführungsformen wird der korrigierte Volumenstrom oder Durchfluss-Wert Fi_korr einer Nachschlagetabelle entnommen, in welcher dieser Wert der Temperaturdifferenz (DT) zugeordnet ist.

In einigen erfindungsgemäßen, beispielhaften Ausführungsformen wird der korrigierte Wert (Fi_korr) folgendermaßen gebildet:

$$(\text{Formel 4}): \quad \text{Fi\_korr} = \text{Fi} * (1 + \text{K} * (\text{DT})),$$

mit

$$i=1 \text{ oder } 2.$$

In manchen erfindungsgemäßen, beispielhaften Ausführungsformen wird der korrigierte Wert (Fi_korr) und/oder eine korrigierte Pumprate oder Fluidrate (oder ein korrigiertes Steuersignal für eine der Pumpen, beispielsweise für die Ultrafiltrationspumpe) wenigstens aus einer, vorzugsweise gespeicherten oder auslesbaren, temperaturabhängigen, physikalischen Größe des Fluids im Fluidkreislauf, beispielsweise seine Dichte, und/oder wenigstens aus einem vorbestimmten, vorzugsweise gespeicherten, auslesbaren oder vorher bestimmbaren, Faktor K ermittelt und/oder berechnet.

In einigen erfindungsgemäßen, beispielhaften Ausführungsformen kann eine korrigierte Ultrafiltrationsrate (UF_korr) basierend wenigstens auf der Temperaturdifferenz (DT) zwischen zwei Volumenströmen wie folgt ermittelt werden:

$$(\text{Formel 5}): \quad \text{UF\_korr} = \text{UF} + \text{F1} * \text{K} + \text{DT}.$$

UF ist dabei die vor erfolgter Korrektur eingestellte Ultrafiltrationsrate.

In bestimmten erfindungsgemäßen, beispielhaften Ausführungsformen können gespeicherte Werte, beispielsweise Werte, welche die Dichte des betroffenen Fluids bei bestimmten Temperaturen angeben, hinterlegt sein und ausgelesen werden. Solche Werte werden in diesen Ausführungsformen zur Berechnung der Fluidbilanz oder eines anderen Wertes, beispielsweise einer korrigierten Ultrafiltrationsrate, verwendet.

In einigen erfindungsgemäßen, beispielhaften Ausführungsformen wird die Fluidbilanz (FB) und/oder die korrigierte Ultrafiltrationsrate als Signal und/oder zur Erzeugung eines Signals für die Steuereinrichtung zum Steuern oder Regeln wenigstens eines Volumenstroms des Fluidkreislaufs verwendet. In manchen erfindungsgemäßen, beispielhaften Ausführungsformen können hierzu insbesondere der erste oder der zweite Volumenstrom erhöht oder erniedrigt werden.

Eine Korrektur kann in manchen erfindungsgemäßen, beispielhaften Ausführungsformen direkt auf elektronische Messwerte aufsetzen. Hierzu werden die aus den erhaltenen Messwerten zu bildenden Signale geeignet korrigiert.

In manchen erfindungsgemäßen, beispielhaften Ausführungsformen wird ein Volumenstrom durch eine Veränderung und/oder Anpassung einer Pumprate wenigstens einer Pumpe, insbesondere des Fluidkreislaufs oder eines hiermit verbundenen Kreislaufs, eines Querschnitts wenigstens eines Abschnitts und/oder einer Temperatur, beispielsweise mittels wenigstens eines Heizers, im Fluidkreislauf, oder in einem verbundenen Kreislauf gesteuert und/oder geregelt werden.

In erfindungsgemäßen, beispielhaften Ausführungsformen ist die Einrichtung zum Erfassen wenigstens einer Temperaturdifferenz zwischen dem ersten und dem zweiten Abschnitt angeordnet und/oder ausgebildet, oder um wenigstens die Temperaturdifferenz zwischen diesen beiden Abschnitten zu erfassen und/oder zu messen.

In manchen erfindungsgemäßen, beispielhaften Ausführungsformen sind die Funktionseinrichtung und/oder wenigstens ein Abschnitt des Fluidkreislaufs vorgesehen und/oder ausgebildet, um mit wenigstens einem Abschnitt wenigstens eines Durchflusssensors, beispielsweise einem Messabschnitt, vorzugsweise lösbar, beispielsweise austauschbar, verbindbar und/oder verbunden zu sein und/oder verbunden zu werden. In bestimmten erfindungsgemäßen, beispielhaften Ausführungsformen liegt eine solche Verbindung vor.

Mit einem "Abschnitt des Fluidkreislaufs" kann in bestimmten, beispielshaften Ausführungsformen insbesondere der erste Abschnitt oder der zweite Abschnitt des Fluidkreislaufs, insbesondere des Dialysat- und/oder Substituatkreislaufs, verstanden werden.

Mit einem "Volumenstrom des Fluidkreislaufs" kann in bestimmten, beispielhaften Ausführungsformen insbesondere der Volumenstrom des ersten Abschnitts oder der Volumenstrom des zweiten Abschnitts des Fluidkreislaufs, insbesondere des Dialysat- und/oder Substituatkreislaufs, verstanden werden.

In einigen erfindungsgemäßen, beispielhaften Ausführungsformen weist die, insbesondere medizinische oder medizintechnische, Funktionseinrichtung wenigstens einen Fluidkreislauf, insbesondere einen Prozessfluidkreislauf, beispielsweise einen Flüssigkeitskreislauf, oder Abschnitte desselben auf.

Der Begriff "Fluidkreislauf", wie er hierin verwendet wird, bezeichnet ein Fluidsystem, einen Schlauchsatz, einen Blutschlauchsatz oder dergleichen, jeweils dazu geeignet, vorgesehen und/oder ausgebildet, um Prozessfluide, medizinische Flüssigkeiten, beispielsweise Blut, Substituat, Dialysat und/oder Kombinationen hiervon, aufzunehmen und von diesen durchströmt zu werden. Die Prozessfluide können zum Zwecke eines Prozesses wie Spülen, Primen, Substituieren, Senken von Schadstoffkonzentrationen und dergleichen zum Einsatz kommen. Ein Fluidkreislauf muss keinen geschlossenen Kreislauf ermöglichen. Ein Fluidkreislauf kann wenigstens eine Filtereinrichtung, beispielsweise einen Blutfilter, und/oder wenigstens eine Pumpe, beispielsweise eine Blut- und/oder eine Substituatpumpe, aufweisen.

In einigen erfindungsgemäßen, beispielhaften Ausführungsformen ist der Fluidkreislauf oder wenigstens ein Abschnitt desselben vorgesehen und/oder ausgebildet zum Aufnehmen und/oder Fördern wenigstens eines Fluids, insbesondere eines medizinischen Fluids.

In einigen erfindungsgemäßen, beispielhaften Ausführungsformen weist die Funktionseinrichtung wenigstens einen Kanal auf, welcher wenigstens dazu geeignet und/oder ausgebildet ist, eine Flüssigkeit zu befördern und/oder zu führen.

In einigen erfindungsgemäßen, beispielhaften Ausführungsformen ist der Fluidkreislauf ein, insbesondere arterieller und/oder venöser, Blut-, Dialysat- und/oder Substituatkreislauf.

Die Funktionseinrichtung kann ferner wenigstens eine Filtereinrichtung aufweisen, welche innerhalb des Fluidkreislaufs, insbesondere zwischen einem Blutkreislauf und wenigstens einem Dialysatkreislauf, oder wenigstens eines Abschnitts hiervon, angeordnet ist. Die Filtereinrichtung kann ein Blutfilter oder Dialysator sein.

In einigen erfindungsgemäßen, beispielhaften Ausführungsformen ist wenigstens ein Abschnitt des Durchflusssensors, vorzugsweise wenigstens ein Messabschnitt hiervon, in der Funktionseinrichtung und/oder im Fluidkreislauf oder in wenigstens einem Abschnitt hiervon, integriert und/oder integral ausgebildet.

In manchen erfindungsgemäßen, beispielhaften Ausführungsformen ist der Durchflusssensor zum Erfassen wenigstens eines Volumenstroms jeweils in oder an wenigstens zwei, vorzugsweise unterschiedlichen, Abschnitten des Fluidkreislaufs ausgebildet.

In einigen erfindungsgemäßen, beispielhaften Ausführungsformen ist der erste Abschnitt in einem Betriebszustand der Funktionseinrichtung, insbesondere in Bezug auf eine Strömungsrichtung des Fluids während des Betriebs der Funktionseinrichtung, beispielsweise während einer Behandlung, einer Filtereinrichtung, beispielsweise einem Blutfilter, vorgeschaltet (stromauf). Alternativ oder ergänzend kann der zweite Abschnitt in einem Betriebszustand der Funktionseinrichtung, insbesondere in Bezug auf eine Strömungsrichtung des Fluids während des Betriebs der Funktionseinrichtung, beispielsweise während einer Behandlung, einer Filtereinrichtung, beispielsweise einem Blutfilter, nachgeschaltet (stromab) sein.

In einigen erfindungsgemäßen, beispielhaften Ausführungsformen können die Funktionseinrichtung und/oder der Fluidkreislauf wenigstens eine Wärmeaustauscheinrichtung aufweisen oder hiermit verbunden sein.

In bestimmten erfindungsgemäßen, beispielhaften Ausführungsformen kann die Wärmeaustauscheinrichtung wenigstens zwischen zwei unterschiedlichen Abschnitten des Fluidkreislaufs, insbesondere zwischen dem ersten Abschnitt und dem zweiten Abschnitt, angeordnet und/oder ausgebildet sein.

In manchen erfindungsgemäßen, beispielhaften Ausführungsformen kann die Wärmeaustauscheinrichtung derart dimensioniert und angeordnet sein, dass die Differenz zwischen der Temperatur des den ersten Abschnitt des Fluidkreislaufs durchströmenden Fluids und der Temperatur des den zweiten Abschnitt des Fluidkreislaufs durchströmenden Fluids innerhalb einer vorbestimmbaren, vordefinierten, einstellbaren und/oder vordefinierbaren, wenigstens begrenzten oder vorbestimmten, Zeit wenigstens reduziert, ausgeglichen, oder beide Temperaturen im Wesentlichen gleich oder identisch sind.

Alternativ oder ergänzend kann in manchen erfindungsgemäßen, beispielhaften Ausführungsformen die Temperaturdifferenz der in den betreffenden Abschnitten durchströmenden Fluide und/oder der jeweiligen Abschnitte vor und nach dem Verlassen der Wärmeaustauscheinrichtung wenigstens reduziert sein, beispielsweise um wenigstens 10%, 20%, 30%, 40%, 50%, 60% oder mehr.

In einigen erfindungsgemäßen, beispielhaften Ausführungsformen ist die Wärmeaustauscheinrichtung insbesondere, bezogen auf die Strömungsrichtung des Fluids in eine Richtung durch die Wärmeaustauscheinrichtung hindurch, wenigstens in einem Abschnitt des Fluidkreislaufs vor wenigstens einem Durchflusssensor im Fluidkreislauf (d. h. stromauf zu diesem) angeordnet und/oder ausgebildet.

In manchen erfindungsgemäßen, beispielhaften Ausführungsformen weist der Fluidkreislauf und/oder die Wärmeaustauscheinrichtung wenigstens einen Heizer auf.

In einigen erfindungsgemäßen, beispielhaften Ausführungsformen ist ein Heizer, insbesondere bezogen auf die Strömungsrichtung des Fluids im Betrieb, vor, an oder nach der Wärmeaustauscheinrichtung, einem Durchflusssensor, einer Filtereinrichtung angeordnet.

In manchen erfindungsgemäßen, beispielhaften Ausführungsformen verläuft wenigstens ein Abschnitt wenigstens eines Durchflusssensors und/oder wenigstens ein Abschnitt des Fluidkreislaufs, insbesondere ein erster und/oder ein zweiter Abschnitt, beispielsweise ein Messabschnitt des Durchflusssensors, durch wenigstens einen Abschnitt der Wärmeaustauscheinrichtung, oder liegt hierin vor.

In bestimmten erfindungsgemäßen, beispielhaften Ausführungsformen ist wenigstens ein Abschnitt des Durchflusssensors und/oder wenigstens ein Abschnitt des Fluidkreislaufs als eingelassener Kanal, vorzugsweise ein im Querschnitt geschlossener oder nicht geschlossener Kanal, etwa ein Halb- oder ein Teilkanal, in der Funktionseinrichtung und/oder in wenigstens einem wärmeleitenden Körper hiervon ausgebildet.

In einigen erfindungsgemäßen, beispielhaften Ausführungsformen kann wenigstens ein Kanal oder Teilkanal von einer Schicht, insbesondere von einer wärmeleitfähigen und/oder fluiddichten Schicht, beispielsweise einer Folie und/oder einer dünnen Platte, nach außen und/oder mittels eines oder gegenüber einem weiteren Kanal abgedichtet sein.

In manchen erfindungsgemäßen, beispielhaften Ausführungsformen weist die erfindungsgemäße Funktionseinrichtung, die erfindungsgemäße Bilanzierungseinrichtung oder die erfindungsgemäße Behandlungsvorrichtung wenigstens eine Auswerteeinheit oder Auswerteeinrichtung zum Bestimmen wenigstens einer Fluidbilanz zwischen wenigstens zwei Abschnitten des Fluidkreislaufs auf, oder ist hiermit in Daten- oder Signalkommunikation verbunden.

In einigen erfindungsgemäßen, beispielhaften Ausführungsformen weist wenigstens die Funktionseinrichtung, die Bilanzierungseinrichtung oder die Behandlungsvorrichtung wenigstens eine Steuer- und/oder Regeleinrichtung (welche im Folgenden vereinfachend als Steuervorrichtung bezeichnet wird, obgleich sie ebenso gut eine Regeleinrichtung sein kann) zum Steuern und/oder Regeln wenigstens eines Volumenstroms in wenigstens einem Abschnitt des Fluidkreislaufs auf.

In gewissen erfindungsgemäßen, beispielhaften Ausführungsformen ist die Steuervorrichtung als Regeleinrichtung ausgestaltet.

In manchen erfindungsgemäßen, beispielhaften Ausführungsformen kann der von einer Steuereinrichtung gesteuerte Volumenstrom insbesondere ein Volumenstrom eines weiteren Abschnitts des Fluidkreislaufs sein, beispielsweise der Volumenstrom, der durch die Filtereinrichtung strömt. Der Volumenstrom kann beispielsweise eine Ultrafiltrationsrate sein.

In einigen erfindungsgemäßen, beispielhaften Ausführungsformen erfolgt die Regelung und/oder Steuerung des Volumenstroms wenigstens auf Basis und/oder wenigstens unter Auswertung der Fluidbilanz, des korrigierten Wertes des Volumenstroms (F2korr, F1korr), der Temperatur oder der Temperaturdifferenz.

In manchen erfindungsgemäßen, beispielhaften Ausführungsformen bilden wenigstens die Auswerteeinrichtung und die Steuereinrichtung eine Einheit.

In einigen erfindungsgemäßen, beispielhaften Ausführungsformen weist wenigstens die Funktionseinrichtung, die Bilanzierungseinrichtung oder die Behandlungsvorrichtung wenigstens einen Durchflusssensor auf oder steht wenigstens in Datenverbindung mit einem solchen.

In einigen erfindungsgemäßen, beispielhaften Ausführungsformen stehen die Auswerteeinrichtung und/oder die Steuereinrichtung wenigstens in Datenverbindung mit wenigstens einem Durchflusssensor, wenigstens einem Temperatursensor und/oder wenigstens einer Behandlungsvorrichtung und/oder jeweils wenigstens Abschnitten hiervon.

In manchen erfindungsgemäßen, beispielhaften Ausführungsformen ist der Durchflusssensor oder wenigstens ein Abschnitt hiervon ein elektromagnetischer Durchflusssensor, insbesondere ein magnetisch-induktiver Durchflusssensor (im Folgenden auch kurz als "MID-Durchflusssensor" bezeichnet) oder weist wenigstens einen solchen auf.

In manchen erfindungsgemäßen, beispielhaften Ausführungsformen ist wenigstens ein Durchflusssensor ein wenigstens zweikanaliger Durchflusssensor oder weist einen solchen auf.

In einigen erfindungsgemäßen, beispielhaften Ausführungsformen ist die Funktionseinrichtung oder die erfindungsgemäße Bilanzierungseinrichtung als Schlauchsystem, Schlauchset, Kassette, insbesondere als Blutkassette, ausgebildet.

In manchen erfindungsgemäßen, beispielhaften Ausführungsformen sind die Funktionseinrichtung und/oder die erfindungsgemäße Bilanzierungseinrichtung als Einwegartikel oder Disposable ausgebildet.

Unter dem Begriff "Einwegartikel oder Disposable", wie er hierhin verwendet wird, kann verstanden werden, dass die medizinische Funktionseinrichtung zur einmaligen Verwendung, beispielsweise in einem Verfahren zum extrakorporalen Behandeln des Bluts eines Patienten, vorgesehen ist. Sie kann als Disposable, als Einwegartikel, als Wegwerfartikel oder dergleichen vorgesehen und/oder vermarktet sein.

In einigen erfindungsgemäßen, beispielhaften Ausführungsformen findet das Bilanzieren in einer Blutbehandlungsvorrichtung oder während einer Blutbehandlung statt. In anderen erfindungsgemäßen Ausführungsformen dagegen nicht.

In bestimmten erfindungsgemäßen, beispielhaften Ausführungsformen steht die Steuer- oder Regelungseinrichtung der erfindungsgemäßen Behandlungsvorrichtung in Signalverbindung mit der erfindungsgemäßen Bilanziereinrichtung. Sie ist konfiguriert und/oder programmiert zur Durchführung wenigstens einer Ausführungsform des erfindungsgemäßen Verfahrens, insbesondere im Zusammenwirken mit den hierzu jeweils erforderlichen Einrichtungen, beispielsweise wie hierin beschrieben.

In einigen erfindungsgemäßen, beispielhaften Ausführungsformen umfasst die Bilanzierungseinrichtung wenigstens eine erfindungsgemäße Funktionseinrichtung oder ist wenigstens in Datenverbindung mit wenigstens einer solchen verbunden und/oder verbindbar.

In manchen erfindungsgemäßen, beispielhaften Ausführungsformen weist die Bilanzierungseinrichtung oder die Funktionseinrichtung oder eine Behandlungsvorrichtung wenigstens eine Auswerteeinheit zum Bilden wenigstens einer Fluidbilanz zwischen wenigstens zwei Volumenströmen in unterschiedlichen Abschnitten eines Fluidkreislaufs auf.

In einigen erfindungsgemäßen, beispielhaften Ausführungsformen weist die Bilanzierungseinrichtung und/oder die Funktionseinrichtung und/oder eine Behandlungsvorrichtung wenigstens eine Auswerteeinrichtung zum Bestimmen wenigstens einer Fluidbilanz zwischen wenigstens zwei Abschnitten des Fluidkreislaufs auf. Alternativ oder ergänzend kann wenigstens die Bilanzierungseinrichtung, die Funktionseinrichtung oder die Behandlungsvorrichtung eine Steuer- und/oder Regeleinrichtung zum Steuern und/oder Regeln wenigstens eines Volumenstroms des Fluidkreislaufs aufweisen.

In bestimmten erfindungsgemäßen, beispielhaften Ausführungsformen sind die Auswerteeinrichtung und/oder die Steuereinrichtung nicht auf der Funktionseinrichtung ausgebildet und/oder angeordnet, sondern können beispielsweise Teil der Bilanzierungseinrichtung oder der Behandlungsvorrichtung sein und/oder an einer Behandlungsvorrichtung angeordnet sein.

Manche, alle oder nur einige der jeweiligen Steuer- und/oder Regeleinrichtungen der Behandlungsvorrichtung, der Bilanzierungseinrichtung und/oder der Funktionseinrichtung können jeweils erfindungsgemäß entsprechende Vorrichtungen, insbesondere zum Durchführen des Verfahrens und/oder zum Steuern und/oder Regeln, wie beispielsweise Blutpumpen, Ultrafiltrationspumpen oder dergleichen, aufweisen. Die entsprechenden Regel- und/oder Steuervorrichtungen können alternativ oder zusätzlich mit diesen Vorrichtungen jeweils wenigstens in Signalverbindung stehen.

Die erfindungsgemäße medizinische Behandlungsvorrichtung ist in bestimmten erfindungsgemäßen Ausführungsformen eine Blutbehandlungsvorrichtung, insbesondere eine Blutreinigungsvorrichtung wie eine Hämodialysiervorrichtung, eine Hämofiltrationsvorrichtung, eine Hämodiafiltrationsvorrichtung, allgemein eine DialyseVorrichtung, beispielsweise eine Vorrichtung zur Akutdialyse, zur Heimdialyse oder zur Peritonealdialyse. Sie kann eine Vorrichtung zum Durchführen von Leberersatzverfahren, eine Vorrichtung zum Durchführen von Immunadsorption oder dergleichen sein.

In manchen erfindungsgemäßen, beispielhaften Ausführungsformen weist die Funktionseinrichtung, die Bilanzierungseinrichtung und/oder die Behandlungsvorrichtung keine Waage und/oder keine Bilanzkammer, insbesondere keine nicht-ideale, starre, Bilanzkammer, auf.

In bestimmten erfindungsgemäßen, beispielhaften Ausführungsformen, weist das Verfahren keine gravimetrische Überwachung auf und/oder die Ultrafiltrationsrate wird nicht direkt mit einem Sensor gemessen, insbesondere nicht mit einem MID-Sensor.

In einigen erfindungsgemäßen, beispielhaften Ausführungsformen weist die Funktionseinrichtung keinen Blutbehälter zur extrakorporalen temporären Speicherung auf, vorzugsweise keinen Blutbehälter, der ein zylinderförmiges Gehäuse aufweist.

In manchen erfindungsgemäßen, beispielhaften Ausführungsformen weist die Funktionseinrichtung keinen Luftabscheider auf.

In einigen erfindungsgemäßen, beispielhaften Ausführungsformen weist die Funktionseinrichtung kein Temperierelement, das in den Luftabscheider integrierbar ist, auf.

In manchen erfindungsgemäßen, beispielhaften Ausführungsformen weist die Funktionseinrichtung kein Peltier-Element auf.

In einigen erfindungsgemäßen, beispielhaften Ausführungsformen weist die Funktionseinrichtung keine Fördereinrichtung oder Fördereinrichtungen auf, insbesondere keine Pumpkammern.

In manchen erfindungsgemäßen, beispielhaften Ausführungsformen weist die Funktionseinrichtung keinen Durchlauferwärmer, insbesondere keine Mehrzahl von Durchlauferwärmern, auf.

Die erfindungsgemäße Funktionseinrichtung weist kein Temperatur-Regelsystem auf, insbesondere keinen elektronischen Regler zum Regeln der Temperatur.

Manche oder alle erfindungsgemäßen Ausführungsformen können einen, mehrere oder alle der oben und/oder im Folgenden genannten Vorteile aufweisen.

Alle oder manche mit dem Verfahren erzielbaren Vorteile lassen sich ungeschmälert in bestimmten Ausführungsformen auch mit der erfindungsgemäßen Funktionseinrichtung, der erfindungsgemäßen Bilanzierungseinrichtung sowie mit der medizinischen Behandlungsvorrichtung erzielen.

Alle mit dem Verfahren erzielbaren Vorteile lassen sich in bestimmten erfindungsgemäßen Ausführungsformen ungeschmälert auch mit den erfindungsgemäßen Vorrichtungen erzielen.

In manchen erfindungsgemäßen Ausführungsformen wird vorteilhaft eine präzise Bilanzierung ermöglicht.

Diese kann vorteilhafterweise zugleich platzsparend und günstig ablaufen.

Während einer extrakorporalen Blutbehandlung werden regelmäßig Heizer oder Heizungen eingesetzt, beispielsweise um Wärmeverluste des Bluts außerhalb des Körpers auszugleichen. Dies kann einen komplexen Temperaturverlauf der verwendeten Flüssigkeiten, insbesondere im extrakorporalen Blutkreislauf, aber auch im restlichen Flüssigkeitskreislauf, verursachen. Somit weisen die zur Bilanzierung herangezogenen zu- und abgeführten Fluide regelmäßig voneinander verschiedenen Temperaturen auf. Da der Temperaturunterschied eine Auswirkung auf das Volumen des Fluids haben kann, kann die Temperatur nicht nur das Volumen sondern auch die Bilanzierung beeinflussen. Diese wird bei fehlender Berücksichtigung der Wirkung der Temperatur auf das Volumen möglicherweise verfälscht.

Messungen mit MID-Durchflusssensoren sind Messungen eines Volumenstroms. Die Dichte des Fluids ist temperaturabhängig. Somit sind der Volumenstrom, sowie ein hierzu korrespondierender Massenstrom, temperaturabhängig. In einigen erfindungsgemäßen Ausführungsformen wird jedoch erreicht, dass die Bilanzierung vorteilhaft unabhängig von der Auswirkung der Temperatur auf das zu bilanzierende Fluid erfolgt.

Weiterhin werden vorteilhafterweise Fehler, die aus einer temperaturabhängigen geometrischen und/oder volumetrischen Veränderung der verwendeten Sensoren resultieren, vorteilhaft vermieden.

In einigen erfindungsgemäßen Ausführungsformen werden vorteilhaft temperaturabhängige Fehler, insbesondere Geometrie- und/oder Dichtefehler, kompensiert.

In manchen erfindungsgemäßen Ausführungsformen kann der durch Temperaturunterschiede verursachte Fehler bei der Bilanzierung wenigstens um den Faktor 10 oder mehr verringert werden.

In manchen erfindungsgemäßen, beispielhaften Ausführungsformen wird vorteilhaft die Messung der Ultrafiltrationsrate vermieden, wodurch insbesondere ein Messsensor für die Messung der Ultrafiltrationsrate entfallen kann.

In einigen erfindungsgemäßen Ausführungsformen, insbesondere bei Ausgestaltungen der Funktionseinrichtung und/oder der Wärmeaustauscheinrichtung als Einwegartikel, kann ein Keimwachstum zwischen aufeinander folgenden Behandlungen unterbrochen werden. Ferner kann das Risiko einer Übertragung einer Infektion von einem Patienten auf den nächsten mit derselben Behandlungsvorrichtung behandelten Patienten weiter gesenkt werden, ohne dass dies besondere Maßnahmen bei der Desinfektion erfordern würde.

In bestimmten erfindungsgemäßen Ausführungsformen kann vorteilhaft auf wenigstens einen Temperatursensor oder auf alle Temperatursensoren verzichtet werden. Ebenso kann das erfindungsgemäße Verfahren mit einem oder ohne jeden Temperatursensor ausgeführt werden.

In den Figuren bezeichnen identische Bezugszeichen gleiche, identische oder ähnliche Komponenten. Es gilt:

**Fig. 1**    zeigt in vereinfachter Darstellung einen Fluidkreislauf einer beispielhaften erfindungsgemäßen Behandlungsvorrichtung;

**Fig. 2**    zeigt in stark vereinfachter Darstellung eine exemplarische erfindungsgemäße Funktionseinrichtung mit zwei Durchflusssensoren;

**Fig. 3**    zeigt in stark vereinfachter Darstellung eine exemplarische Anordnung einer Behandlungsvorrichtung mit einer weiteren erfindungsgemäßen Funktionseinrichtung;

**Fig. 4**    zeigt in stark vereinfachter Darstellung eine weitere erfindungsgemäße Funktionseinrichtung; und

**Fig. 5**    zeigt in stark vereinfachter Darstellung eine wiederum weitere erfindungsgemäße Funktionseinrichtung.

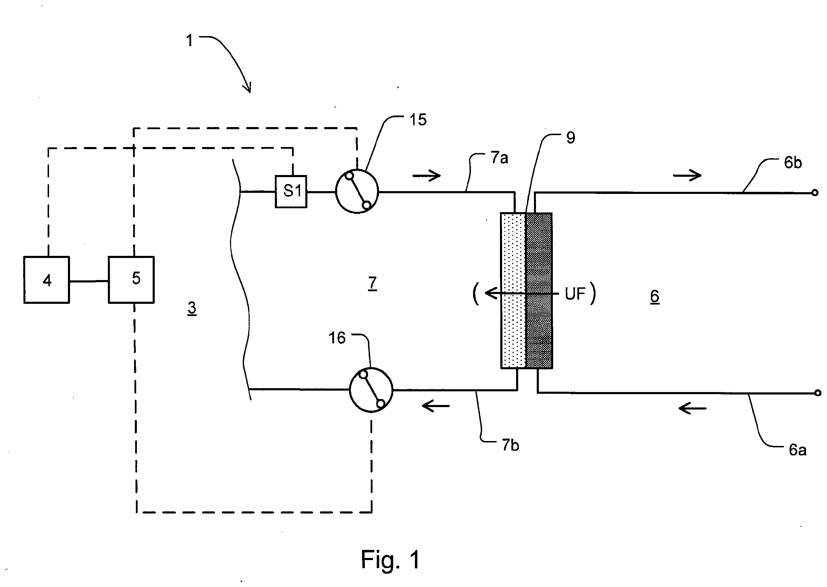

**Fig. 1** zeigt in stark vereinfachter Darstellung einen Abschnitt eines Fluidkreislaufs 1, welcher mit einer nur angedeuteten erfindungsgemäßen Behandlungsvorrichtung 3 in Fluidverbindung verbunden ist oder zumindest abschnittsweise Teil der Behandlungsvorrichtung 3 ist. Die Behandlungsvorrichtung 3 ist mit einer erfindungsgemäßen Bilanzierungseinrichtung 4 verbunden oder weist diese auf. Die Behandlungsvorrichtung 3 ist zudem mit einer Steueroder Regeleinrichtung 5 zum Steuern oder Regeln von Pumpen, Ventilen, usw. zum Durchführen der Behandlung des Patienten verbunden oder weist diese auf.

Die Bilanzierungseinrichtung 4 ist wenigstens in Datenverbindung mit einer Einrichtung S1 zum Erfassen der Temperatur.

Die Bilanzierungseinrichtung 4 kann Teil der Steuer- oder Regeleinrichtung 5 der Behandlungsvorrichtung 3 sein, oder mit erster in Signalverbindung stehen wie beispielsweise in Fig. 1 gezeigt.

Die in Fig. 1 und allen folgenden Figuren dargestellte Behandlungsvorrichtung 3 ist rein exemplarisch als Ultrafiltrationsvorrichtung ausgestaltet, der Fluidkreislauf 1 als Kreislauf für die extrakorporale Blutbehandlung. Beide dieser Annahmen dienen allein dem besseren Verständnis der vorliegenden Erfindung anhand eines konkreten Beispiels und sollen die Erfindung keinesfalls beschränken.

Der Fluidkreislauf 1 weist einen extrakorporalen Blutkreislauf 6 und einen Dialysatkreislauf 7 auf, welche mittels einer Filtereinrichtung 9 rein exemplarisch sowohl im Stoff- als auch im Fluidaustausch miteinander stehen. Die Pfeile bezeichnen eine Strömungsrichtung der verschiedenen Flüssigkeiten während einer Behandlung eines nicht gezeigten Patienten mittels der Behandlungsvorrichtung 3.

Im extrakorporalen Blutkreislauf 6 fließt Blut des Patienten durch eine arterielle Blutschlauchleitung 6a zur Filtereinrichtung 9, um darin gereinigt zu werden. Durch eine venöse Blutschlauchleitung 6b wird das gereinigte Blut zum Patienten zurückgeführt.

Im Dialysatkreislauf 7, welcher ein Substituat- oder Dialysatkreislauf sein kann und eine Dialysierflüssigkeitsleitung 7a und eine Dialysatleitung 7b aufweist, sind vereinfachend eine erste Pumpe 15 und eine zweite Pumpe 16 zum Erzeugen eines bestimmten Volumenstroms im Dialysatkreislauf 7 angeordnet. Diese stehen, wie mit der Strichlinie angedeutet, mit der Steuer- oder Regeleinrichtung 5 jeweils in Signalkommunikation.

Auch der Blutkreislauf 6 weist vorzugsweise wenigstens eine in den Figuren nicht gezeigte Pumpe zum Fördern von Blut auf.

In Fig. 1 zeigt ein Pfeil eine rein optional erfolgende Ultrafiltration an, bei welcher Fluid aus dem Blutkreislauf 6 mit einem Durchfluss oder Volumenstrom UF (Ultrafiltrationsrate) über den Filter 9 in den Dialysatkreislauf 7 eintritt. Die Ultrafiltrationsrate UF kann mittels der Steuer- oder Regeleinrichtung 5 und entsprechender Pumpen, etwa der ersten und/oder zweiten Pumpe 15, 16, eingestellt werden.

Die Anordnung der Fig. 1 ist aufgrund der entsprechend programmierten Bilanzierungseinrichtung 4 und/oder der entsprechend programmierten Steuer- und Regeleinrichtung 5 geeignet, das erfindungsgemäße Verfahren auszuführen.

Die in Fig. 1 gezeigten Pumpen 15, 16 sind der Einfachheit halber in den folgenden Figuren nicht erneut dargestellt. Sie können jedoch unverändert auch in den folgenden beschriebenen Ausführungsformen vorgesehen sein.

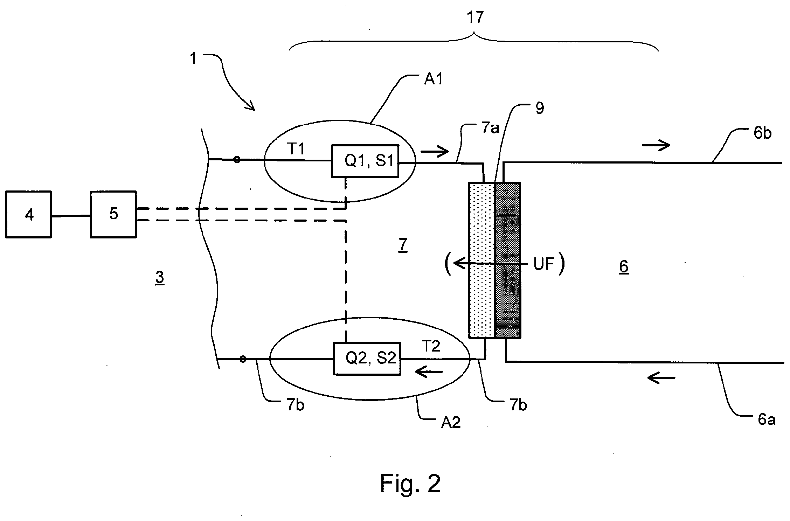

**Fig. 2** zeigt in stark vereinfachter Darstellung eine erfindungsgemäße Funktionseinrichtung 17, welche Teile des Dialysatkreislaufs 7 und rein optional auch Teile des Blutkreislaufs 6 umfasst. Die Funktionseinrichtung 17 weist üblicherweise nicht den Filter 9 auf; sie kann hiermit jedoch in Fluidkommunikation verbunden oder verbindbar sein.

In einem ersten Abschnitt A1 des Dialysatkreislaufs 7 weist letzterer einen ersten Durchflusssensor Q1 auf oder ist hiermit verbunden. In einem zweiten Abschnitt A2 weist der Dialysatkreislauf 7 wenigstens einen zweiten Durchflusssensor Q2 auf oder ist hiermit verbunden. Der erste und/oder der zweite Durchflusssensor Q1, Q2 sind optional Teil der erfindungsgemäßen Funktionseinrichtung 17, der Bilanzierungseinrichtung 4 oder der Behandlungsvorrichtung 3.

Der erste Abschnitt A1 liegt beispielsweise in der Dialysierflüssigkeitsleitung 7a, der zweite Abschnitt A2 beispielsweise in der Dialysatleitung 7b.

Die Temperatur des in den ersten Abschnitt A1 einströmenden Fluids wird als T1 bezeichnet.

Die Temperatur des Fluids, welches aus dem zweiten Abschnitt A2 ausströmt, wird als T2' bezeichnet. Der zweite Abschnitt A2 ist optional stromab der Filtereinrichtung 9 angeordnet.

Die Temperatur des Fluids, welches in den zweiten Abschnitt A2 einströmt, wird als T2 bezeichnet.

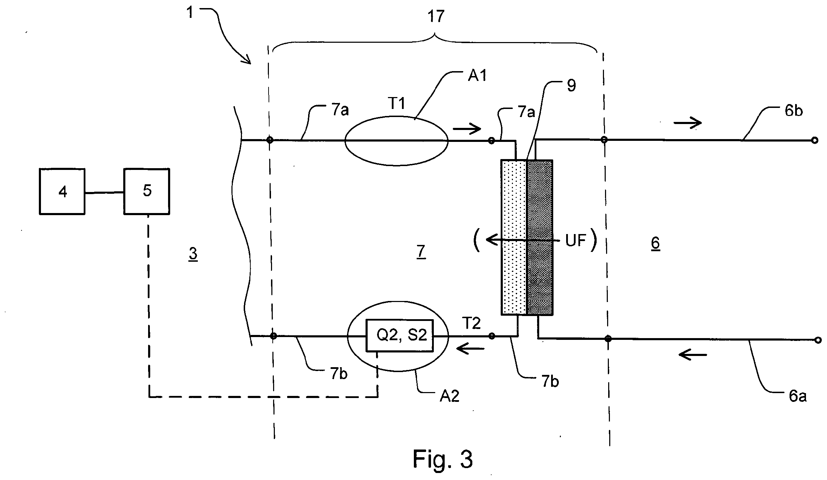

Die Temperatur T1 kann mittels eines rein optional vorgesehenen Temperatursensors S1 ermittelt werden. Wie Fig. 3 zu entnehmen ist, ist dieser Temperatursensor S1 bei Kenntnis oder Schätzung der Temperatur T1 jedoch nicht erforderlich.

Die Temperatur T2 kann mittels eines ebenfalls rein optional vorgesehenen Temperatursensors S2 ermittelt werden. Dieser Temperatursensor S2 ist bei Kenntnis oder Schätzung der Temperatur T2 nicht erforderlich. Beispielsweise kann in einigen Behandlungsszenarien in ausreichend guter Näherung angenommen werden, dass T2 der Körpertemperatur des Patienten entspricht.

Anstelle des Temperatursensors S1 und/oder des Temperatursensors S2 kann der Fluidkreislauf 1 eine Einrichtung zum Erfassen der Temperaturdifferenz zwischen dem ersten und dem zweiten Abschnitt aufweisen.

Wie in Fig. 2 mittels Strichlinie angedeutet, stehen die Sensoren Q1, Q2, S1, S2 vorzugsweise mit der Steuer- oder Regeleinrichtung 5 in Signalverbindung.

**Fig. 3,** die - wie auch die folgenden Figuren - auf Fig. 1 und Fig. 2 aufbaut, zeigt in stark vereinfachter Darstellung eine erfindungsgemäße Funktionseinrichtung 17 in einer weiteren beispielhaften Ausführungsform.

Die Funktionseinrichtung 17 entspricht im Wesentlichen jener der Fig. 2. Jedoch ist hier anders als bei der Ausführungsform der Fig. 2 kein Temperatursensor S1 zur Messung der Temperatur T1 vorgesehen, jedenfalls wird die Temperatur T1 nicht mittels eines Temperatursensors S1 gemessen. Der Temperatursensor S1 bzw. sein Einsatz sind vielmehr bei Kenntnis oder Schätzung der Temperatur T1 entbehrlich. Beispielsweise kann in einigen Behandlungsszenarien in ausreichend guter Näherung angenommen werden, dass T1 jener Temperatur entspricht, mit welcher das Fluid, das den ersten Abschnitt A1 durchströmt, jener Temperatur entspricht, mit welcher das Fluid in einer Fluidquelle vorliegt.

Es sei ferner angemerkt, dass in bestimmten Ausführungen der Temperatursensor S2 zum Messen der Temperatur T2 nicht zwingend erforderlich ist, jedenfalls muss die Temperatur T2 nicht in jedem Fall mittels eines Temperatursensors S2 gemessen werden. Tatsächlich zeigt beispielsweise gebrauchtes Dialysat beim Verlassen der Filtereinrichtung 9 eine geringe Temperaturschwankung. Regelmäßig bewegt sich die Temperatur der aus der Filtereinrichtung 9 ausströmenden Flüssigkeit im Bereich der Körpertemperatur (ca. 37°C), so dass daher erfindungsgemäß vorgesehen sein kann, zum Bestimmen der Temperaturdifferenz DT ausschließlich die Temperatur der einströmenden Flüssigkeit im ersten Abschnitt A1 zu bestimmen oder gar keine Temperatur zu bestimmen (sondern diese anzunehmen), anders als in Fig. 3 gezeigt.

Fig. 3 zeigt ferner, dass auch der erste Durchflusssensor Q1 entbehrlich sein kann. Dies gilt jedenfalls dann, wenn der erste Durchfluss F1 bekannt oder geschätzt werden kann. Dies kann beispielsweise der Fall sein, wenn wenigstens der erste Volumenstrom F1 oder der zweite Volumenstrom F2, insbesondere mittels einer hierfür geeigneten Vorrichtung, beispielsweise eine Pumpe und/oder eine Steuer- und/oder Regeleinrichtung und/oder eine Eingabevorrichtung, einstellbar und/oder eingestellt ist.

Das besondere Merkmal der Ausführungsform der Fig. 3, nach welchem es keines Durchflusssensors Q1 und/oder keines Temperatursensors S1 für den ersten Abschnitt A1 und/oder keines Temperatursensors S2 für den zweiten Abschnitt A2 bedarf, ist mit jeder anderen Ausgestaltung der vorliegenden Erfindung, wo sinnvoll, kombinierbar.

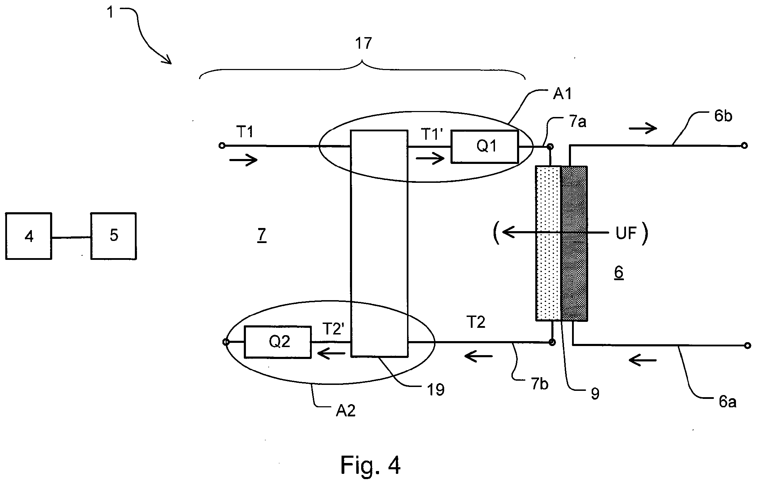

**Fig. 4** zeigt in stark vereinfachter Darstellung eine weitere erfindungsgemäße Ausführungsform der Funktionseinrichtung 17.

Die Funktionseinrichtung 17 weist optional einen ersten und einen zweiten Durchflusssensor Q1 und Q2 auf. Sie weist zudem wenigstens eine Wärmeaustauscheinrichtung 19 auf oder ist hiermit verbunden.

Der optional vorgesehene erste Durchflusssensor Q1 ist in einem ersten Abschnitt A1 des Substituats- oder Dialysatkreislaufs 7 des Fluidkreislaufs 1 angeordnet, vorzugsweise in der Dialysierflüssigkeitsleitung 7a. Der optional vorgesehene zweite Durchflusssensor Q2 ist in einem zweiten Abschnitt A2 des Substituats- oder Dialysatkreislaufs 7 des Fluidkreislaufs 1 angeordnet, vorzugsweise in der Dialysatleitung 7b.

Der erste Abschnitt A1 liegt rein optional stromauf einer Filtereinrichtung 9 (beispielsweise einem Blutfilter), der zweite Abschnitt A2 liegt rein optional stromab der Filtereinrichtung 9. In diesem Beispiel liegen der erste und der zweite Abschnitt A1, A2 optional jeweils mit der Filtereinrichtung 9 in Fluidverbindung.

Die Wärmeaustauscheinrichtung 19 ist entlang der Strömungsrichtung des Fluids während der Blutbehandlung, welche mittels Pfeilen angedeutet ist, für einen Austausch von Wärme oder thermischer Energie zwischen dem Fluid, welches den ersten Abschnitt A1 durchströmt, und dem Fluid, welches den zweiten Abschnitt A2 durchströmt, angeordnet.

Insbesondere ist die Wärmeaustauscheinrichtung 19 optional stromauf des ersten Durchflusssensors Q1 angeordnet, welcher stromauf der Filtereinrichtung 9 angeordnet ist. Die Wärmeaustauscheinrichtung 19 ist zugleich optional stromauf des zweiten Durchflusssensors Q2 angeordnet, welcher stromab der Filtereinrichtung 9 angeordnet ist.

Die Wärmeaustauscheinrichtung 19 kann optional Teil des ersten und/oder des zweiten Abschnitt A1, A2 sein, insbesondere ein integrales Teil hiervon, und/oder optional mit dem ersten und/oder mit dem zweiten Abschnitt A1, A2 nur, insbesondere thermisch, verbunden sein.

Die Wärmeaustauscheinrichtung 19 ist vorgesehen, geeignet und/oder ausgebildet, um einen Wärmeaustausch und vorzugsweise sogar einen Wärmeausgleich zwischen dem Fluid, das den ersten Abschnitt A1 durchströmt, und dem Fluid, das den zweiten Abschnitt A2 durchströmt, zu bewirken.

Ein Wärmeausgleich kann bedeuten, dass die Differenz zwischen einer Temperatur T1' des Fluids nach Verlassen der Wärmaustauschvorrichtung 19 aber vor dem ersten Durchflusssensor Q1, und insbesondere vor der Filtereinrichtung 9, und einer Temperatur T2' der ausströmenden Flüssigkeit nach dem Durchgang der Wärmaustauschvorrichtung 19 aber vor dem zweiten Durchflusssensor Q2 Null oder im Wesentlichen Null ist. Unter einem Wärmeausgleich kann erfindungsgemäß auch zu verstehen sein, dass vorstehend genannte Differenz jedenfalls kleiner als die Differenz zwischen den Temperaturen T1 und T2 ist, welche das Fluid oder die Fluide jeweils vor Eintritt in die Wärmaustauschvorrichtung 19 aufwies.

Obwohl die Ausführung der Fig. 4 keine Temperatursensoren benötigt ist erfindungsgemäß optional zusätzlich vorgesehen, die Temperatur T1 und/oder die Temperatur T2, beispielsweise mittels wenigstens eines Temperatursensors (nicht dargestellt), zu erfassen und/oder, insbesondere mittels einer geeigneten Einrichtung wie einem Heizer, einzustellen.

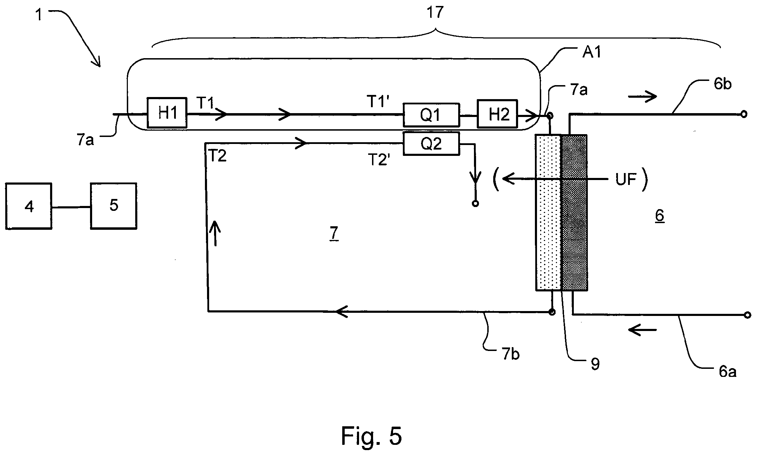

**Fig. 5** zeigt in stark vereinfachter Darstellung die Funktionseinrichtung 17 noch in einer wiederum weiteren erfindungsgemäßen Ausführungsform. Dabei kann die Anordnung und/oder Ausführung der Funktionseinrichtung 17 zusätzlich wenigstens einen Heizer Hi (i=1, 2) aufweisen.

Der Heizer Hi kann insbesondere im ersten Abschnitt A1 des Fluidkreislaufs 1 angeordnet sein.

Im dargestellten Beispiel sind zwei Heizer H1 und H2 dargestellt. Beide sind beispielhaft im ersten Abschnitt A1 angeordnet und/oder ausgebildet. Beispielsweise ist der erste Heizer H1 dem Durchflusssensor Q1 vorgelagert, während der zweite Heizer H2 beispielsweise dem Durchflusssensor Q1 nachgelagert ist. Rein exemplarisch sind beide Heizer H1, H2 nicht Teil der Funktionseinrichtung 17. Sie könnten dies jedoch - zumindest abschnittsweise - sein.

Es kann erfindungsgemäß vorgesehen sein, nur einen Heizer, H1 oder H2, oder weitere Heizer im Fluidkreislauf 7 vorzusehen.

Es kann auch vorgesehen sein, dass wenigstens ein Heizer Hi, insbesondere in einem ersten Abschnitt A1 des Fluidkreislaufs 7, einer Wärmeaustauscheinrichtung 19 vorgelagert ist und/oder einem der Wärmeaustauscheinrichtung 19 nachgelagerten Durchflusssensor nachgelagert ist (nicht dargestellt).

In einer weiteren, nicht dargestellten Ausführungsform der Funktionseinrichtung 17 kann ein Heizer Hi rein optional an, in und/oder wenigstens in Verbindung mit einer der Wärmeaustauscheinrichtung 19, ausgebildet und/oder angeordnet sein (nicht dargestellt).

Der Heizer kann ausgebildet sein, um eine vorbestimmte Temperatur des Fluids zu gewährleisten. Somit kann vorteilhaft in bestimmten Ausführungsformen vollständig auf eine Temperaturmessung verzichtet werden.

Heizer, wie sie in Fig. 5 gezeigt sind, können selbstredend auch mit den Anordnungen der in den vorangegangenen Figuren gezeigten Ausführungsformen kombiniert werden.

Mittels der Anordnung einer der Figuren und der besonderen Ausgestaltung der Bilanzierungseinrichtung 4 kann eine korrekte Fluidbilanz FB erzeugt werden. Alternativ oder ergänzend kann mittels der Steuervorrichtung 5 eine korrigierte Ultrafiltrationsrate UF_korr erzeugt werden.

Im Folgenden wird die vorliegende Erfindung mittels der folgenden Beispiele und zum Teil unter Bezugnahme auf die vorstehend diskutierten Figuren weiter beschrieben. Die folgenden Beispiele sind, wie auch alle in den Beispielen angegebenen Zahlenwerte, rein exemplarisch und stellen keine Beschränkung der Erfindung dar.

In den ersten Abschnitt A1, siehe beispielsweise Fig. 2, strömt mit T1 = 20°C eine Flüssigkeit ein, welche - rein exemplarisch - eine Dialysierflüssigkeit sein kann. Der erste Durchflusssensor Q1 misst einen Durchfluss F1 von 200 ml/min.

Im zweiten Abschnitt A2 strömt Dialysat mit T2 = 37°C. Dort misst der zweite Durchflusssensor Q2 einen Durchfluss F2 von 200 ml/min.

Temperaturabhängige Fehler, verursacht beispielsweise durch eine Dichtedifferenz der Flüssigkeit und/oder Volumendifferenzen des Fluids an den beiden Durchflusssensoren Q1, Q2, welche sich aufgrund der Temperaturdifferenz des Fluids zwischen A1 und A2 ergeben, können in einem Kreislauf wie dem Fluidkreislauf 7 bis zu 0,5% des Messergebnisses (hier der Fluss F1 oder F2) oder mehr betragen. In einem solchen beispielhaften Fall können dem Patient daher unbemerkt beispielsweise 60 ml/h Flüssigkeit zu wenig entzogen werden.

Erfindungsgemäß ermöglicht das Verfahren vorteilhaft, solche temperaturabhängigen Fehler, welche insbesondere geometrische (d. h. Veränderungen des Lumens des Schlauchs und andere) und/oder Dichtefehler sein können, beispielsweise wie folgt beschrieben zu berücksichtigen und/oder zu kompensieren.

Beispiel 1:

In manchen erfindungsgemäßen Ausführungsformen wird zu diesem Zweck die Temperaturdifferenz DT ermittelt, also die Differenz zwischen den Temperaturen T1 und T2, welche das Fluid an den beiden Messstellen S1 bzw. S2 im ersten Abschnitt A1 bzw. zweiten Abschnitt A2 aufweist.

In Kenntnis der Temperaturdifferenz DT kann der gemessene Volumenstromwert, hier beispielsweise der Durchfluss oder Volumenstrom F2, gemessen mittels des zweiten Durchflusssensors Q2, rechnerisch korrigiert werden. Dabei kann die rechnerische Korrektur proportional zur Temperaturdifferenz und/oder zu einem Faktor K, hier beispielsweise K = 0,000294/°C (pro Grad Celsius) erfolgen.

Der Faktor K kann experimentell und/oder theoretisch ermittelt werden und beispielsweise in einer Tabelle vorliegen. Ebenso kann der Korrekturwert in einer Tabelle vorgehalten werden.

Die Temperaturdifferenz DT beträgt in diesem Beispiel:

$$DT = T2-T1 = (37-20)°C = 17°C.$$

Der korrigierte Wert des zweiten Durchflusssensors kann aus Formel 3' folgendermaßen errechnet werden:

$$F2\_korr = F2 * (1 + K * DT) = 200 * (1 + 0,000294 * 17)$$
$$= 201 \; ml/min.$$

In diesem Beispiel kann der gemessene Volumenstromwert F2 nun durch den zweiten Volumenstromwert F2_korr erfindungsgemäß ersetzt werden. Diese Korrektur, welche zu einer Erhöhung des rechnerisch weiter verwendeten Messwerts des zweiten Durchflusssensors Q2 um 1 ml/min führt, erlaubt es, die Fluidbilanz FB durch Verändern eines Behandlungsparameters auszugleichen (so dass sie Null wird oder ihre Abweichung unter einen vorbestimmten Schwellenwert liegt). Ein Eingriff in die Steuerung der Behandlungsvorrichtung ist hier nicht erforderlich, kann aber vorgenommen werden, wie Beispiel 2 zeigt.

Beispiel 2:

Ausgehend von einem System wie bereits zu Beispiel 1 erläutert, wird hier zusätzlich eine Filtereinrichtung 9 wie in Fig. 2 dargestellt, angenommen. Sie wird angesteuert, um beispielsweise eine Ultrafiltrationsrate (UF) von 500 ml/h zu bewirken.

Wie im Beispiel 1 liegt der temperaturabhängige Fehler, insbesondere der Dichtefehler, in der Größenordnung von ca. 0,5% des Flüssigkeitsflusses.

Ohne Kompensation der temperaturabhängigen Fehler und mit den Werten des vorherigen Beispiels 1 würde daher durch die Filtereinrichtung eine effektive Ultrafiltrationsrate von 440 ml/h zustande kommen anstelle der erwünschten Ultrafiltrationsrate (UF) von 500 ml/h.

Erfindungsgemäß kann eine korrigierte Ultrafiltrationsrate (UF_korr), die es einzustellen gilt, basierend auf der Temperaturdifferenz (DT) zwischen den zwei Volumenströmen wie folgt ermittelt werden:

```
UF_korr   = UF + F1 * K + DT
          = 500 ml/h + (200 ml/h * 0,000294 * 17)
          = 560 ml/h.
```

Die korrigierte Ultrafiltrationsrate UF_korr kann erfindungsgemäß zur Steuerung und/oder Regelung einer Steuer- und/oder Regeleinrichtung, beispielsweise einer Ultrafiltrationspumpe, übermittelt und/oder verwendet werden.

## **Bezugszeichenliste**

| | |
|---|---|
| 1 | Fluidkreislauf |
| 3 | Behandlungsvorrichtung |
| 4 | Bilanzierungseinrichtung |
| 5 | Steuer- und/oder Regeleinrichtung |
| 6 | Blutkreislauf |
| 6a | arterielle Blutschlauchleitung |
| 6b | venöse Blutschlauchleitung |
| 7 | Dialysat- und/oder Substituatkreislauf |
| 7a | Dialysierflüssigkeitsleitung |
| 7b | Dialysatleitung |
| 9 | Filtereinrichtung |
| 15 | erste Pumpe |
| 16 | zweite Pumpe |
| 17 | Funktionseinrichtung |
| 19 | Wärmeaustauscheinrichtung |
| A1 | erster Abschnitt des Dialysat- und/oder Substituatkreislaufs |
| A2 | zweiter Abschnitt des Dialysat- und/oder Substituatkreislaufs |
| Q1 | erster Durchflusssensor |
| Q2 | zweiter Durchflusssensor |
| S1 | Temperatursensor |
| S2 | Temperatursensor |
| F1 | erster Volumenstrom im ersten Abschnitt des Fluidkreislaufs |
| F2 | zweiter Volumenstrom im zweiten Abschnitt des Fluidkreislaufs |
| T1 | erste Temperatur in wenigstens einem ersten Abschnitt des Fluidkreislaufs |
| T1' | erste Temperatur in wenigstens einem ersten Abschnitt des Fluidkreislaufs nach Durchgang der Wärmeaustauscheinrichtung |
| T2 | zweite Temperatur in wenigstens einem zweiten Abschnitt des Fluidkreislaufs |
| T2' | zweite Temperatur in wenigstens einem zweiten Abschnitt des Fluidkreislaufs nach Durchgang der Wärmeaustauscheinrichtung |
| T | Temperatur in wenigstens einem Abschnitt des Fluidkreislaufs |

**Patentansprüche**

1.  Medizinische Behandlungsvorrichtung (3) zum Behandeln eines medizinischen Fluids, konfiguriert zum Ausführen eines Verfahrens zum Bestimmen wenigstens einer Fluidbilanz (FB) zwischen wenigstens einem ersten Volumenstrom (F1) eines ersten Abschnitts (A1) eines Fluidkreislaufs (1) einer medizinischen Funktionseinrichtung (17) der medizinischen Behandlungsvorrichtung und wenigstens einem zweiten Volumenstrom (F2) eines zweiten Abschnitts (A2) des Fluidkreislaufs (1) der medizinischen Funktionseinrichtung (17), und/oder zum Steuern und/oder Regeln wenigstens eines Volumenstroms (F1, F2, UF) wenigstens eines Abschnitts des Fluidkreislaufs (1) der medizinischen Funktionseinrichtung (17), umfassend wenigstens folgende Schritte:

    - Einstellen, Messen und/oder Annehmen wenigstens eines ersten Volumenstroms (F1) in wenigstens einem ersten Abschnitt (A1) des Fluidkreislaufs (1);
    - Einstellen, Messen und/oder Annehmen wenigstens einer ersten Temperatur (T1) in wenigstens einem ersten Abschnitt (A1) des Fluidkreislaufs (1) und Messen wenigstens einer zweiten Temperatur (T2) in wenigstens einem zweiten Abschnitt (A2) des Fluidkreislaufs (1); oder Messen wenigstens einer Temperaturdifferenz (DT) zwischen dem ersten und dem zweiten Abschnitt (A2) des Fluidkreislaufs (1);
    - Messen wenigstens eines zweiten Volumenstroms (F2) in wenigstens einem zweiten Abschnitt (A2) des Fluidkreislaufs (1); und
    - Bilden einer Fluidbilanz (FB) aus wenigstens dem ersten Volumenstrom (F1) und einem korrigierten Wert des zweiten Volumenstroms (F2_korr), wobei der korrigierte Wert (F2_korr) wenigstens aus dem zweiten Volumenstrom (F2) und der zweiten Temperatur (T2) und/oder der Temperaturdifferenz (DT) ermittelt und/oder berechnet wird,

    wobei der zweite Volumenstrom durch einen Durchflusssensor gemessen wird.

2.  Medizinische Behandlungsvorrichtung (3) nach Anspruch 1, wobei der erste Volumenstrom durch einen Durchflusssensor gemessen wird.

3.  Medizinische Behandlungsvorrichtung (3) nach Anspruch 1 oder 2, wobei die Fluidbilanz (FB), der korrigierte Wert des Volumenstroms (F2_korr), die Temperatur (T1) oder die Temperaturdifferenz (DT) als Signal und/oder zur Erzeugung eines Signals für eine Steuer- und/oder eine Regeleinrichtung (5) zum Steuern und/oder Regeln wenigstens eines Volumenstroms (F1, F2, UF) des Fluidkreislaufs (1), einer Pumprate wenigstens einer Pumpe des Fluidkreislaufs (1), eines Querschnitts wenigstens eines Abschnitts des Fluidkreislaufs (1) und/oder einer Temperatur im Fluidkreislauf verwendet werden.

4.  Medizinische Behandlungsvorrichtung (3) nach einem der vorhergehenden Ansprüche, wobei der korrigierte Wert (F2_korr) des zweiten Volumenstroms (F2) wenigstens eine gespeicherte, temperaturabhängige, physikalische Größe des Fluids im Fluidkreislauf (1) und/oder einen vorbestimmten Faktor K berücksichtigt.

5.  Medizinische Funktionseinrichtung (17) konfiguriert zur Verwendung mit der medizinischen Behandlungsvorrichtung (3) nach einem der Ansprüche 1 bis 4 und mit wenigstens einem Fluidkreislauf (1) oder einem Abschnitt hiervon, welche wenigstens einen ersten und einen zweiten Durchflusssensor (Q1, Q2) aufweist, wobei der jeweilige Durchflusssensor (Q1, Q2) zum Messen wenigstens eines Volumenstroms (F1, F2) in wenigstens einem ersten bzw. einem zweiten Abschnitt (A1, A2) des Fluidkreislaufs (1) ausgebildet und/oder angeordnet ist, wobei die Funktionseinrichtung (17) wenigstens eine Einrichtung zum Messen einer Temperatur (T) in dem ersten Abschnitt (A1) und dem zweiten Abschnitt (A2) des Fluidkreislaufs (1) oder zum Messen der Temperaturdifferenz (DT) zwischen dem ersten und dem zweiten Abschnitt (A1, A2) aufweist, wobei die Durchflusssensoren (Q1, Q2) als magnetisch-induktive Durchflusssensoren oder als ein Abschnitt hiervon ausgebildet sind, wobei die medizinische Funktionseinrichtung (17) kein Temperatur-Regelsystem, insbesondere keinen elektronischen Regler zum Regeln der Temperatur, aufweist.

6.  Funktionseinrichtung (17) nach Anspruch 5, wobei der Durchflusssensor (Q1, Q2) wenigstens als zweikanaliger Durchflusssensor ausgebildet ist.

7.  Funktionseinrichtung (17) nach einem der vorangehenden Ansprüche 5 und 6, wobei die Funktionseinrichtung (17) als Schlauchsystem und/oder als Kassette ausgebildet ist.

8.  Funktionseinrichtung (17) nach einem der Ansprüche 5 bis 7, wobei die Funktionseinrichtung (17) oder der Fluidkreislauf (1) wenigstens eine Wärmeaustauscheinrichtung (19) aufweist oder hiermit verbunden ist oder verbindbar ist, welche zwischen dem ersten Abschnitt (A1) des Fluidkreislaufs (1) und dem zweiten Abschnitt (A2) des Fluidkreislaufs (1) angeordnet und/oder ausgebildet ist.

9. Funktionseinrichtung (17) nach einem der vorangehenden Ansprüche 5 bis 8, welche als Einwegartikel oder Disposable ausgebildet ist.

10. Bilanzierungseinrichtung (4) zum Bestimmen wenigstens einer Fluidbilanz zwischen wenigstens oder genau einem ersten Volumenstrom (F1) in einem ersten Abschnitt (A1) eines Fluidkreislaufs (1) und einem zweiten Volumenstrom (F2) in einem zweiten Abschnitt (A2) des Fluidkreislaufs (1), wobei die Bilanzierungseinrichtung (4) wenigstens eine Funktionseinrichtung (17) nach einem der Ansprüche 5 bis 9 aufweist.

11. Bilanzierungseinrichtung (4) nach Anspruch 10, umfassend wenigstens eine Auswerteeinheit zum Bilden wenigstens einer Fluidbilanz (FB) zwischen wenigstens einem oder genau einem ersten Volumenstrom (F1) in einem ersten Abschnitt (A1) eines Fluidkreislaufs (1) und einem zweiten Volumenstrom (F2) in einem zweiten Abschnitt (A2) des Fluidkreislaufs (1) und/oder eine Steuer- und/oder Regeleinrichtung (5) zum Steuern und/oder Regeln wenigstens eines Volumenstroms (F1, F2, UF) des Fluidkreislaufs (1).

**Claims**

1. A medical treatment apparatus (3) for treating a medical fluid, configured for carrying out a method for determining at least one fluid balance (FB) between at least one first volume flow (F1) of a first section (A1) of a fluid circuit (1) of a medical functional device (17) of the medical treatment apparatus and at least one second volume flow (F2) of a second section (A2) of the fluid circuit (1) of the medical functional device (17), and/or for controlling and/or regulating at least one volume flow (F1, F2, UF) of at least one section of the fluid circuit (1) of the medical functional device (17), comprising at least the following steps:

   - setting, measuring and/or assuming at least one first volume flow (F1) in at least one first section (A1) of the fluid circuit (1);
   - setting, measuring and/or assuming at least one first temperature (T1) in at least one first section (A1) of the fluid circuit (1), and measuring at least one second temperature (T2) in at least one second section (A2) of the fluid circuit (1); or measuring at least one temperature difference (DT) between the first and the second section (A2) of the fluid circuit (1);
   - measuring at least one second volume flow (F2) in at least one second section (A2) of the fluid circuit (1); and
   - forming a fluid balance (FB) from at least the first volume flow (F1) and one corrected value of the second volume flow (F2_korr), the corrected value (F2_korr) being determined and/or calculated at least from the second volume flow (F2) and the second temperature (T2) and/or the temperature difference (DT),

   wherein the second volume flow is measured by a flow sensor.

2. The medical treatment apparatus (3) according to claim 1, wherein the first volume flow is measured by a flow sensor.

3. The medical treatment apparatus (3) according to claim 1 or 2, wherein the fluid balance (FB), the corrected value of the volume flow (F2_korr), the temperature (T1) or the temperature difference (DT) are used as signal and/or for generating a signal for a control and/or a regulating device (5) for controlling and/or regulating at least a volume flow (F1, F2, UF) of the fluid circuit (1), a pump rate of at least one pump of the fluid circuit (1), a cross-section of at least one section of the fluid circuit (1) and/or a temperature in the fluid circuit.

4. The medical treatment apparatus (3) according to anyone of the preceding claims, wherein the corrected value (F2_korr) of the second volume flow (F2) takes into account at least one stored, temperature-dependent, physical property of the fluid in the fluid circuit (1) and/or a predetermined factor K.

5. A medical functional device (17) configured for use with the medical treatment apparatus (3) according to anyone of claims 1 to 4 and having at least one fluid circuit (1), or one section thereof, comprising at least a first and a second flow sensor (Q1, Q2), wherein the respective flow sensor (Q1, Q2) is designed and/or arranged for measuring at least one volume flow (F1, F2) in at least a first or a second section (A1, A2) of the fluid circuit (1), wherein the functional device (17) comprises at least one means for measuring a temperature (T) in the first section (A1) and the second section (A2) of the fluid circuit (1) or for measuring the temperature difference (DT) between the first and the second section (A1, A2), wherein the flow sensors (Q1, Q2) are designed as magnetic-inductive flow sensors or as a section thereof,

wherein the medical functional device (17) does not have a temperature regulating system, in particular no electronic regulator for regulating the temperature.

6.  The functional device (17) according to claim 5, wherein the flow sensor (Q1, Q2) is at least embodied as a two-channel flow sensor.

7.  The functional device (17) according to anyone of the preceding claims 5 and 6, wherein the functional device (17) is embodied as a tubing system and/or as a cassette.

8.  The functional device (17) according to anyone of the claims 5 to 7, wherein the functional device (17) or the fluid circuit (1) comprises at least one heat exchange device (19), or is connected or connectable therewith, which is arranged and/or embodied between the first section (A1) of the fluid circuit (1) and the second section (A2) of the fluid circuit (1).

9.  The functional device (17) according to anyone of the preceding claims 5 to 8, which is embodied as a single-use item or as a disposable.

10. A balancing unit (4) for determining at least one fluid balance between at least, or precisely, one first volume flow (F1) in a first section (A1) of a fluid circuit (1) and a second volume flow (F2) in a second section (A2) of the fluid circuit (1), wherein the balancing unit (4) comprises at least one functional device (17) according to anyone of claims 5 to 9.

11. The balancing unit (4) according to claim 10, comprising at least one evaluation unit for generating at least one fluid balance (FB) between at least one, or precisely, one first volume flow (F1) in a first section (A1) of a fluid circuit (1) and a second volume flow (F2) in a second section (A2) of the fluid circuit (1), and/or comprising a control and/or regulating device (5) for controlling and/or regulating at least one volume flow (F1, F2, UF) of the fluid circuit (1).

## Revendications

1.  Un appareil de traitement médical (3) destiné à traiter un fluide médical, configuré pour exécuter un procédé pour déterminer au moins un bilan fluidique (FB) entre au moins un premier débit volumique (F1) d'une première partie (A1) d'un circuit fluidique (1) d'un dispositif fonctionnel médical (17) de l'appareil de traitement médical ainsi qu'au moins un second débit volumique (F2) d'une seconde partie (A2) du circuit fluidique (1) du dispositif fonctionnel médical (17), et/ou pour commander et/ou réguler au moins un débit volumique (F1, F2, UF) d'au moins une partie du circuit fluidique (1) du dispositif fonctionnel médical (17), comprenant au moins les étapes suivantes:

    - le réglage, la mesure et/ou l'assomption d'au moins un premier débit volumique (F1) dans au moins une première partie (A1) du circuit fluidique (1);
    - le réglage, la mesure et/ou l'assomption d'au moins une première température (T1) dans au moins une première partie (A1) du circuit fluidique (1) ainsi que la mesure d'au moins une seconde température (T2) dans au moins une seconde partie (A2) du circuit fluidique (1); ou la mesure d'au moins une différence de température (DT) entre la première et la seconde partie (A2) du circuit fluidique (1);
    - la mesure d'au moins un second débit volumique (F2) dans au moins une seconde partie (A2) du circuit fluidique (1) ; et
    - la formation d'un bilan fluidique (FB) d'après au moins le premier débit volumique (F1) ainsi qu'une valeur corrigée du second débit volumique (F2_korr), la valeur corrigée (F2_korr) étant déterminée et/ou calculée au moins d'après le second débit volumique (F2) ainsi que la seconde température (T2) et/ou la différence de température (DT),

    où le second débit volumique est mesuré par un débimètre.

2.  L'appareil de traitement médical (3) selon la première revendication, où le premier débit volumique est mesuré par un débimètre.

3. L'appareil de traitement médical (3) selon la revendication 1 ou 2, où le bilan fluidique (FB), la valeur corrigée du débit volumique (F2_korr), la température (T1) ou la différence de température (DT) sont utilisé(e)s comme signal et/ou pour générer un signal destiné à un dispositif de commande et/ou de régulation (5) afin de commander et/ou de réguler au moins un débit volumique (F1, F2, UF) du circuit fluidique (1), un taux de pompage d'au moins une pompe du circuit fluidique (1), une section transversale d'au moins une partie du circuit fluidique (1) et/ou une température dans le circuit fluidique.

4. L'appareil de traitement médical (3) selon l'une quelconque des revendications précédentes, où la valeur corrigée (F2_korr) du second débit volumique (F2) prend en compte au moins une grandeur physique du fluide sauvegardée, dépendant de la température, dans le circuit fluidique (1) et/ou un facteur K prédéterminé.

5. Un dispositif fonctionnel médical (17) configuré de façon à être utilisé avec l'appareil de traitement médical (3) selon l'une quelconque des revendications 1 à 4 et ayant au moins un circuit fluidique (1), ou une partie de celui-ci, comprenant au moins un premier ainsi qu'un second débimètres (Q1, Q2), où le débimètre respectif (Q1, Q2) est conçu et/ou agencé de façon à mesurer au moins un débit volumique (F1, F2) dans au moins une première, respectivement dans une seconde partie (A1, A2) du circuit fluidique (1), où le dispositif fonctionnel (17) comprend au moins un dispositif pour mesurer une température (T) dans la première partie (A1) ainsi que dans la seconde partie (A2) du circuit fluidique (1), ou pour mesurer la différence de température (DT) entre la première et la seconde partie (A1, A2), où les débimètres (Q1, Q2) sont conçus comme des débimètres magnéto-inductifs ou comme une partie de ceux-ci, où le dispositif fonctionnel médical (17) ne comprend pas de système de régulation de la température, notamment pas de régulateur électronique pour réguler la température.

6. Le dispositif fonctionnel (17) selon la revendication 5, où le débimètre (Q1, Q2) est au moins conçu comme un débimètre à deux canaux.

7. Le dispositif fonctionnel (17) selon l'une quelconque des revendications 5 et 6 précédentes, où le dispositif fonctionnel (17) est conçu comme un système de tuyaux et/ou comme une cassette.

8. Le dispositif fonctionnel (17) selon l'une quelconque des revendications 5 à 7, où le dispositif fonctionnel (17) ou le circuit fluidique (1) comprend au moins un dispositif d'échange thermique (19), ou est relié, ou peut être relié à celui-ci, qui est agencé et/ou conçu entre la première partie (A1) du circuit fluidique (1) et la seconde partie (A2) du circuit fluidique (1).

9. Le dispositif fonctionnel (17) selon l'une quelconque des revendications 5 à 8 précédentes, qui est conçu comme un article à usage unique ou comme un article jetable.

10. Un dispositif d'équilibrage (4) destiné à déterminer au moins un bilan fluidique entre au moins, ou précisément, un premier débit volumique (F1) dans une première partie (A1) d'un circuit fluidique (1) ainsi qu'un second débit volumique (F2) dans une seconde partie (A2) du circuit fluidique (1), où le dispositif d'équilibrage (4) comprend au moins un dispositif fonctionnel (17) selon l'une quelconque des revendications 5 à 9.

11. Le dispositif d'équilibrage (4) selon la revendication 10, comprenant au moins une unité d'évaluation pour générer au moins un bilan fluidique (FB) entre au moins un, ou précisément, un premier débit volumique (F1) dans une première partie (A1) d'un circuit fluidique (1) ainsi qu'un second débit volumique (F2) dans une seconde partie (A2) du circuit fluidique (1) et/ou comprenant un dispositif de commande et/ou de régulation (5) pour commander et/ou réguler au moins un débit volumique (F1, F2, UF) du circuit fluidique (1).

Fig. 1

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4530759 A
- EP 0715859 A